# EUROPEAN PATENT APPLICATION

(11) **EP 2 407 183 A1**
(43) Date of publication of application: **18.01.2012**
(21) Application number: 10750345.0
(22) Date of filing: 08.03.2010
(51) Int. Cl.: A61L 27/04, C23C 14/48, A61L 31/02, C23C 14/14

(54) **DEGRADABLE STENT**

(30) Priority: 10.03.2009 CN 200910058554
(71) Applicant: Southwest Jiaotong University, Sichuan 610031 (CN)
(72) Inventor: HUANG, Nan, Sichuan 610031 (CN); LENG, Yongxiang, Sichuan 610031 (CN); YANG, Ping, Sichuan 610031 (CN); SUN, Hong, Sichuan 610031 (CN); WANG, Jin, Sichuan 610031 (CN); CHEN, Junying, Sichuan 610031 (CN); WAN, Guojiang, Sichuan 610031 (CN); JING, Fengjuan, Sichuan 610031 (CN); ZHAO, Ansha, Sichuan 610031 (CN); XIONG, Kaiqin, Sichuan 610031 (CN); YOU, Tianxue, Sichuan 610031 (CN); WU, Xi, Sichuan 610031 (CN)
(74) Representative: Zhao, Yi
(86) International application number: PCT/CN2010/070905
(87) International publication number: WO 2010/102549

(57) **Abstract**

Degradable pure iron stent or iron alloy stent containing 0.01 to 0.5 atom% of La, Ce or Sr are surface modified using ion implantation or plasma ion implantation to implant oxygen, nitrogen, La, Ce or Sr into the stent surface, or to deposit a thin film of La, Ce, Sr, lanthana, ceria, strontia, iron or iron oxide onto the stent surface. The thickness of the deposited films is from 10 to 1000 nanometers with the grain size from 10 to 200 nanometers. The corrosion resistance of these stents is significantly increased, and the stents have good biocompatibility. The degradation of the stents is controllable. The stents can also provide sufficient support in blood vessel in 3-6 months after intervention and be degraded after 6 months.

## Description

### TECHNICAL FIELD

This present invention relates to biodegradable stents applied in treating vascular and brain blood vessel diseases.

### BACKGROUND ART

Percutaneous transluminal coronary angioplasty (PTCA) has been applied in clinic since 1977. PTCA is delivering a balloon into target lesion position of blood vessel through femoral artery, then applying a pressure to expand the balloon so that the inside dimension of the narrowed vascular can be increased to improve the blood supplying to the cardiac muscle. However the restenosis rate after PTCA is as high as more than 50%. The application of metal stents made of stainless steel, cobalt alloy or nickel titanium alloy can decrease the restenosis rate to about 20% to 30%. Since 2003, drug eluting stent (DES) coated with a polymer layer containing drugs can decease the restenosis rate to about 10%, DES is regarded as a milestone of interventional therapy of coronary heart diseases. However, all these stents are not degradable and will stay in the blood vessel permanently. There is a big difference in mechanical characteristics between the blood vessel and the stent, which may cause chronic damage of blood vessel, atrophy of middle layer of the blood vessel, aneurysm formation and endometrial hyperplasia. Furthermore, young patients who are still in the growth period, the permanent stent cannot meet the need of the growth of the blood vessel. Simultaneously, the harmful elements released from stents to blood vessel are also the issue of metal stents.

An ideal stent should perform the function of efficient mechanical support after intervention into the target lesion blood vessel in an appropriate period of 3 to 6 months, simultaneously release the drug to realize the function of therapy, and after that time, be gradually degraded to decrease the lesion effect of the stent on the blood vessel to prevent restenosis. Therefore, biodegradable polymer stent, such as polylactic acid stent, has attracted much attention. Polylactic acid can be degraded to non-toxic water and CO₂ which will be absorbed by the human body or breathed out, it has been approved by FDA as a biomaterial on the market. Completely biodegradable polymer stent which releases drugs during degrade process has also been reported. H. Tamai et al reported their results of intervention of 25 pieces of polylactic acid stents into hearts of 15 patients. Their results show that after 6 months the restenosis rate was at the same level of stainless steel stent. However, there are still problems with biodegradable polymer stents, such as:
1) The strength and the deformation behavior of polymer stents are much different from metal stents. Because the biodegradable polymer stent is not strong, the thickness of polylactic acid stent is double that of stainless steel stent to provide suitable support, which will bring about obstructing to the blood stream.
2) The polymer stents have to be heated to expand sufficiently, while heating may easily damage the blood vessel.
3) The rebound rate of polymer stents is high, and visibility of the polymer is poor inside the vessel.
4) The delivery system for biodegradable polymer stent is different from the delivery system which is widely used presently for metal stents. This would also affect the application of polymer stents.

Biodegradable metal stents do not have those issues with polymer stents. Therefore, it is important to develop biodegradable metal stent and stent made of iron (Fe) is a new research subject. Iron is a common element existing in human body. The total amount of iron in an adult body is about 4 gram, and an adult will need to incept 10 to 15 mg of iron every day. Iron has better biocompatibility and mechanical properties. In 2001, Peuster firstly reported in vivo results of intervention of 16 iron stents (Fe content > 99.8%) into abdominal aorta of New Zealand rabbits for 6 to 18 months, most of the support bars of the stents degraded to some degree after 6 months and no obvious inflammation, restenosis and coagulation were found in 6 to 18 months experiments. [Peuster M, Wohlsein P, Brugmann M, et al, A novel approach to temporary stenting: degradable cardiovascular stents produced from corrodible metal-results 6 18 months after implantation into New Zealand white rabbits. Heart, 2001;86:563-569]

Peuster further intervened stainless steel stents and iron stents into the abdominal aorta of mini-pigs in 2006. It was found that the degree of endometrial hyperplasia of both kinds of stents was about the same, and no excessive deposition and toxicity effect on the main organs of the pigs were found after histopathology analysis of these organs. And no toxic and side effects caused by corrosion product were found in the tissues close to the iron stent ribs.

In 2006, Mueller et al used gene chip technology to reveal that one of the degradation products of pure iron stent, Fe²⁺, has an effect to inhibit the proliferation of smooth muscle cell. In 2008, Waksman et al intervened iron stents and Co-Cr alloy stents into pig's conorary blood vessel for 28 days and found that no obvious inflammation and embolism on surfaces of iron stents. And no obvious difference was found in intima thickness, intima area and occlusion rate between the two kinds of stents.

In terms of strength, plasticity and processing ability, iron stent is close to stainless steel stent. Iron stent has better mechanical supporting ability than polymer stent. Comparing with Co-Cr alloy stent or stainless steel stent, iron stent has degradation ability and a better biocompatibility. The clinic applied balloon delivery system is also suitable for iron stent, it is easy for operation, and its cost is low.

However there are still some shortcomings in iron stent: the low corrosion resistance of pure iron stent surface which can affect its mechanical support and be unhelpful to recovery of the lesion position of blood vessel, the low covering rate of endothelial cells on the stent surface in short time after the intervention into blood vessel, and the degradation rate of the iron stent which cannot be regulated to meet different needs.

### SUMMARY OF THE INVENTION

The present invention is about a degradable stent with a high corrosion resistivity and controllable degradation. In the 30 days after the stent intervened into blood vessel, the degradation rate of the stent maintains a low level and endue the surface with a good biocompatibility, which will be beneficial for endothelial cells to grow and cover the stent surface. And the stent can maintain a good mechanical supporting ability during 3 to 6 months and be degraded after 6 months.

One part of the present invention is about pure iron stents and treated by surface modification using ion implantation technique, as follows:
Implanting oxygen or nitrogen ions into the pure iron stent surface by ion implantation or plasma immersion ion implantation, the doses range is from 1×10¹⁶ to 5×10¹⁸ atoms/cm², the energy range of the ions is from 5 to 100 KeV.

Or implanting lanthanum (La), cerium (Ce) or strontium (Sr) ions into the pure iron stent surface by ion implantation or plasma immersion ion implantation, the doses range is from 1×10¹⁶ to 5×10¹⁸ atoms/cm², the energy range of the ions is from 5 to 100 KeV.

Or depositing a thin film of La, Ce, Sr, lanthana, ceria, strontia, iron or iron oxide on the pure iron stent using plasma immersion ion implantation. The thickness of the films is from 10 to 1000 nanometers, the grain size is from 10 to 200 nanometers.

Comparing with existing technologies, the present invention of stents with O, N, La, Ce, Sr, lanthana, ceria, strontia, iron, iron oxide modified surface using ion implantation and/or plasma immersion ion implantation increases surface corrosion resistance of the stent. The elements for surface modification are non-toxic. The degradation rate of the iron stent can be effectively controlled and modulated. The stent maintains a low degradation rate in the 30 days after the intervention and endue the surface with a good biocompatibility which will be beneficial for endothelial cells to grow and cover the stent surface. The stent will provide sufficient mechanical supporting in 3 to 6 months period after intervention, and be degraded and gradually disappear after 6 months. Thus it will not have the issue of restenosis and late- thrombus formation etc. which may be caused by no-degradable permanent stent. The experiment results prove that the degradation rate of surface modified iron stent can go down more than 50% comparing with non- surface modified iron sten. Endothelial cells grow on the stent surface within 4 weeks, while on non- surface modified iron stent no endothelial cells existed but only degradation product. The degree of the degradation rate of iron stent can be modulated by controlling the dosage of implanted ions, energy of the ion implantion, thickness or grain sizes of the deposited thin films to meet the different needs of the stent.

Above mentioned iron stent can be further magnetized in a magnetic field to become magnetic. The magnetized stent has the advantages to promote endothelial cells covering and prohibit smooth muscle cells growth, which is beneficial for prohibiting restenosis and coagulation. The mechanical supporting ability of the stent in 3 to 6 months period after intervention is sufficient, and the stent will be degraded and gradually disappear after 6 months.

The other part of the present invention is fabricating of iron alloy stent. The iron alloy contains 0.01 to 0.5% of La, Ce or Sr. By adjusting the contents of La, Ce or Sr, the corrosion resistance/ degradation rate of the stent can be modulated. The stent maintains a low degradation rate in the first 30 days after the intervention and endue the surface with a good biocompatibility which will be beneficial for endothelial cells to grow and cover the stent surface. The stent will provide sufficient mechanical supporting in 3 to 6 months period after intervention, and be degraded and gradually disappear after 6 months. Thus it will not have the issue of restenosis and late- thrombus formation etc. which may be caused by no-degradable permanent stent. The experiment results prove that the degradation rate of the iron alloy stent can go down more than 50% in the first 30 days after being intervened into blood vessel, comparing with pure iron stent. The degradation rate of iron alloy stent can be modulated by controlling quantity of alloying elements to meet different needs of the stent.

Above mentioned iron alloy stent is further treated by surface modification using ion implantation technique to further improve the surface corrosion resistance of stent and modulate the degradation rate. The surface modification treatment is as follows:
Implanting oxygen or nitrogen ions into the iron alloy stent surface by ion implantation or plasma immersion ion implantation, the doses range is from 1×10¹⁶ to 5×10¹⁸ atoms/cm², the energy range of the ions is from 5 to 100 KeV.

Or implanting lanthanum (La), cerium (Ce) or strontium (Sr) ions into the iron alloy stent surface by ion implantation or plasma immersion ion implantation, the doses range is from 1×10¹⁶ to 5×10¹⁸ atoms/cm², the energy range of the ions is from 5 to 100 KeV.

Or depositing a thin film of La, Ce, Sr, lanthana, ceria, strontia, iron or iron oxide on the iron alloy stent using plasma immersion ion implantation. The thickness of the films is from 10 to 1000 nanometers, the grain size is from 10 to 200 nanometers.

Above mentioned iron alloy stent can be further magnetized in a magnetic field. The magnetized stent has advantages to promote endothelial cells covering and prohibit smooth muscle cells growth, which can be beneficial to prohibit restenosis and coagulation.

The invention is further demonstrated by the figures and the examples, in comparison of controlling samples of pure iron stent:

### BRIEF DESCRIPTION OF THE DRAWINGS

Fig. 1 is a SEM micrograph of surface morphology of the stent described in example 5 intervened into dog's femoral artery for 4 weeks.
Fig. 2 is a SEM micrograph of surface morphology of untreated pure iron stent intervened into dog's femoral artery for 4 weeks.
Fig. 3 is surface morphology of the stent in example 95 with iron oxide film cultured with endothelial cells for 3 days.
Fig.4 is surface morphology of the stainless steel stent cultured with endothelial cells for 3 days.

### DETAILED DESCRIPTION OF THE INVENTION

The following EXAMPLES illustrate various aspects of the making the stent invention herein. They are not intended to limit the scope of the invention.

### EXAMPLE 1 to 9

A biodegradable pure iron stent is treated by the following plasma processes:
Oxygen ions are implanted into the pure stent surface by ion implantation or plasma immersion ion implantation, the doses range is from 1×1016 to 5×1018 atoms/cm2, the energy range of the ions is from 5 to 100 KeV. The treatment parameters and test results of the example 1-9 are given in Table 1.

For verifying the corrosion resistance and in vitro degradation properties of the stents, polarization testing and immersion corrosion testing are performed using simulated body fluid solution (SBF, containing NaCl:8.04, KCl:0.23, NaHCO3:0.35, K2HPO4-3H2O:0.24, MgCl2·6H2O:0.31, CaCl2:0.29, Na2SO4:0.07, TRIS:6.12). The results are given in Table 1. It is proved that the corrosion currents and the weight loss are all significantly decreased. Less weight loss also means a more stable mechanical supporting of the stent.

**Table 1**

| | method | Implanted doses (atoms/cm²) | Ion energy (KeV) | Corrosion current (mA/cm²) | Weight loss after immersion 216 hours (mg) |
|---|---|---|---|---|---|
| Controlling sample | Unmodified iron stent | | | 0.59 | 0.460 |
| Example 1 | Ion implantation | 1×10¹⁶ | 5 | 0.40 | 0.340 |
| Example 2 | Plasma immersion ion implantation | 1×10¹⁶ | 40 | 0.45 | 0.390 |
| Example 3 | Ion implantation | 1×10¹⁶ | 100 | 0.49 | 0.416 |
| Example 4 | Plasma immersion ion implantation | 1×10¹⁷ | 5 | 0.27 | 0.258 |
| Example 5 | Plasma immersion ion implantation | 1×10¹⁷ | 40 | 0.08 | 0.092 |
| Example 6 | Ion implantation | 1×10¹⁷ | 100 | 0.12 | 0.112 |
| Example 7 | Plasma immersion ion implantation | 5×10¹⁸ | 5 | 0.36 | 0.306 |
| Example 8 | Ion implantation | 5×10¹⁸ | 40 | 0.13 | 0.121 |
| Example 9 | Plasma immersion ion implantation | 5×10¹⁸ | 100 | 0.11 | 0.105 |

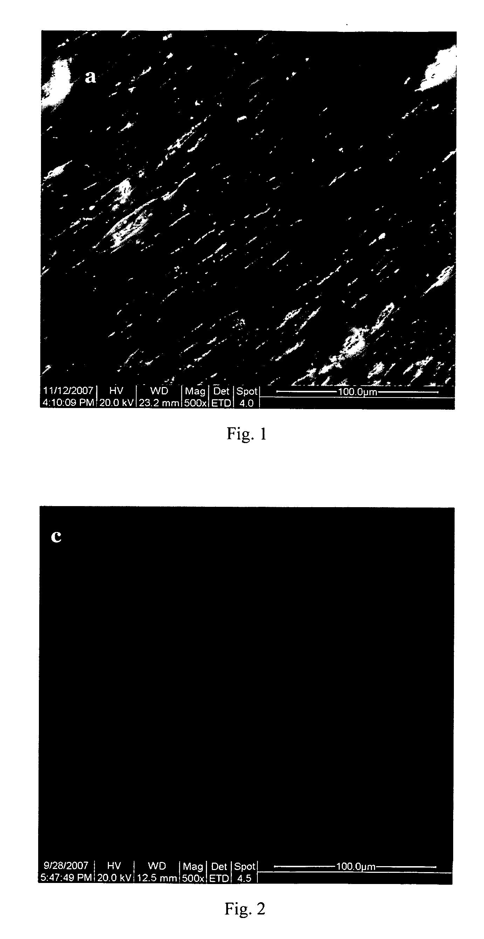

Fig. 1 is a SEM micrograph of surface morphology of the stent described in example.5 intervened into a dog's femoral artery for 4 weeks. Fig. 2 is a SEM micrograph of surface morphology of the controlling sample of untreated pure iron stent intervened into the dog's femoral artery for 4 weeks. Comparing Fig. 1 with Fig.2, it shows that after oxygen ions implantation, endothelial cells have covered on the modified iron stent completely, but almost no endothelial cells grew on the untreated iron stent. This result indicates that biocompatibility of the oxygen ions implanted iron stent is significantly improved.

### EXAMPLE 10 to 18

A biodegradable pure iron stent is treated by the following processes:
Nitrogen ions are implanted into the pure iron stent surface by ion implantation or plasma immersion ion implantation, the doses range is from 1×10¹⁶ to 5×10¹⁸ atoms/cm², the energy range of the ions is from 5 to 100 KeV. The parameters for example 10-18 are given in Table 2.

Table 2 also shows the corrosion and in vitro degradation properties of the stents in these examples by polarization testing and immersion corrosion testing. It is proved that the corrosion resistance of the surface modified stent is significantly improved as showed by the significant lower weight loss after immersed in SBF for 9 days. And the lowest weight loss is 1/3 of that of the untreated one.

**Table 2**

| | Surface modification method | Implanted doses (atoms/cm²) | Ion energy (KeV) | Corrosion current (mA/cm²) | Weight loss after immersion 216 hours (mg) |
|---|---|---|---|---|---|
| Controlling sample | Unmodified iron stent | | | 0.59 | 0.460 |
| example 10 | Ion implantation | 1×10¹⁶ | 5 | 45 | 0.415 |
| example 11 | Plasma immersion ion implantation | 1×10¹⁶ | 40 | 0.51 | 0.438 |
| example 12 | Ion implantation | 1×10¹⁶ | 100 | 0.53 | 0.445 |
| example 13 | Plasma immersion ion implantation | 1×10¹⁷ | 5 | 0.35 | 0.318 |
| example 14 | Plasma immersion ion implantation | 1×10¹⁷ | 40 | 0.18 | 0.163 |
| example 15 | Ion implantation | 1×10¹⁷ | 100 | 0.22 | 0.239 |
| example 16 | Plasma immersion ion implantation | 5×10¹⁸ | 5 | 0.41 | 0.352 |
| example 17 | Plasma immersion ion implantation | 5×10¹⁸ | 40 | 0.20 | 0.169 |
| example 18 | Ion implantation | 5×10¹⁸ | 100 | 0.18 | 0.158 |

### EXAMPLE 19 to 27

A biodegradable pure iron stent is treated by the following processes:
La ions are implanted into the pure iron stent surface by ion implantation or plasma immersion ion implantation, the doses range is from 1×10¹⁶ to 5×10¹⁸ atoms/cm², the energy range of the ions is from 5 to 100 KeV. The parameters for example 19-27 are given in Table 3.

Table 3 also gives the results of polarization testing, immersion corrosion testing and activated partial thromboplastin time (APTT). It is proved that corrosion current and weight loss are significantly decreased and APTT is increased. These results show that La ion implantation modifies the blood compatibility, corrosion resistance and supporting ability of the iron stent.

**Table 3**

| | Surface modification method | Implanted doses (atoms/cm²) | Ion energy (KeV) | Corrosion current (mA/cm²) | Weight loss after immersion 216 hours (mg) | APTT(s) |
|---|---|---|---|---|---|---|
| Controlling sample | Untreated pure iron | | | 0.57 | 0.481 | 39.5 |
| Example 19 | Ion implantation | 1×10¹⁶ | 5 | 0.38 | 0.316 | 41.5 |
| Example 20 | Plasma immersion ion implantation | 1×10¹⁶ | 40 | 0.41 | 0.342 | 41.0 |
| Example 21 | Ion implantation | 1×10¹⁶ | 100 | 0.44 | 0.373 | 42.5 |
| Example 22 | Plasma immersion ion implantation | 1×10¹⁷ | 5 | 0.24 | 0.234 | 41.5 |
| Example 23 | Ion implantation | 1×10¹⁷ | 40 | 0.20 | 0.191 | 42.0 |
| Example 24 | Ion implantation | 1×10¹⁷ | 100 | 0.25 | 0.238 | 42.0 |
| Example 25 | Plasma immersion ion implantation | 5×10¹⁸ | 5 | 0.34 | 0.288 | 42.5 |
| Example 26 | Plasma immersion ion implantation | 5×10¹⁸ | 40 | 0.24 | 0.226 | 41.0 |
| Example 27 | Ion implantation | 5×10¹⁸ | 100 | 0.22 | 0.202 | 42.5 |

### EXAMPLE 28 to 36

A biodegradable pure iron stent is treated by the following processes:
Ce ions are implanted into the pure iron stent surface by ion implantation or plasma immersion ion implantation, the doses range is from 1×10¹⁶ to 5×10¹⁸ atoms/cm², the energy range of the ions is from 5 to 100 KeV. The parameters for example 28-36 are given in Table 4.

Table 4 also gives the results of polarization testing, immersion corrosion testing and activated partial thromboplastin time (APTT). It is proved that the corrosion current and the weight loss is significantly decreased and APTT is increased. These results show that Ce ion implantation modified the blood compatibility, corrosion resistance and supporting ability of the iron stent.

**Table 4**

| | Surface modification method | Implanted doses (atoms/cm²) | Ion energy (KeV) | Corrosion current (mA/cm²) | Weight loss after immersion 216 hours (mg) | APTT(s) |
|---|---|---|---|---|---|---|
| Controlling sample | Unmodified pure iron | | | 0.58 | 0.481 | 39.7 |
| Example 28 | Ion implantation | 1×10¹⁶ | 5 | 0.41 | 0.337 | 41.2 |
| Example 29 | Plasma immersion ion implantation | 1×10¹⁶ | 40 | 0.38 | 0.309 | 42.6 |
| Example 30 | Ion implantation | 1×10¹⁶ | 100 | 0.47 | 0.399 | 41.0 |
| Example 31 | Plasma immersion ion implantation | 1×10¹⁷ | 5 | 0.27 | 0.248 | 42.0 |
| Example 32 | Plasma immersion ion implantation | 1×10¹⁷ | 40 | 0.19 | 0.177 | 43.5 |
| Example 33 | Ion implantation | 1×10¹⁷ | 100 | 0.23 | 0.217 | 41.8 |
| Example 34 | Plasma immersion ion implantation | 5×10¹⁸ | 5 | 0.37 | 0.301 | 42.5 |
| Example 35 | Plasma immersion ion implantation | 5×10¹⁸ | 40 | 0.28 | 0.238 | 41.5 |
| Example 36 | lonimplantation | 5×10¹⁸ | 100 | 0.26 | 0.221 | 43.0 |

### EXAMPLE 37 to 45

A biodegradable pure iron stent is treated by the following processes:
Sr ions are implanted into the pure iron stent surface by ion implantation or plasma immersion ion implantation, the doses range is from 1×10¹⁶ to 5×10¹⁸ atoms/cm², the energy range of the ions is from 5 to 100 KeV. The parameters for example 37-45 are given in Table 5.

Table 5 also gives the results of polarization testing, immersion corrosion testing and activated partial thromboplastin time (APTT). It is proved that the corrosion current and the weight loss is significantly decreased and APTT is increased. These results show that Sr ion implantation modified the blood compatibility, corrosion resistance and supporting ability of the iron stent.

**Table 5**

| | Surface modification method | Implanted doses (atoms/cm²) | Ion energy (KeV) | Corrosion current (mA/cm²) | weight loss after immersion 216 hours (mg) | APTT(s) |
|---|---|---|---|---|---|---|
| Controlling sample | Unmodified pure iron | | | 0.56 | 0.466 | 40.0 |
| Example 37 | Ion implantation | 1×10¹⁶ | 5 | 0.37 | 0.305 | 42.5 |
| Example 38 | Plasma immersion ion implantation | 1×10¹⁶ | 40 | 0.32 | 0.278 | 43.0 |
| Example 39 | Ion implantation | 1×10¹⁶ | 100 | 0.41 | 0.357 | 41.5 |
| Example 40 | Plasma immersion ion implantation | 1×10¹⁷ | 5 | 0.28 | 0.248 | 43.5 |
| Example 41 | Plasma immersion ion implantation | 1×10¹⁷ | 40 | 0.23 | 0.227 | 43.8 |
| Example 42 | Ion implantation | 1×10¹⁷ | 100 | 0.31 | 0.269 | 42.5 |
| Example 43 | Plasma immersion ion implantation | 5×10¹⁸ | 5 | 0.35 | 0.294 | 42.6 |
| Example 44 | Plasma immersion ion implantation | 5×10¹⁸ | 40 | 0.22 | 0.214 | 42.2 |
| Example 45 | Ion implantation | 5×10¹⁸ | 100 | 0.21 | 0.193 | 41.5 |

### EXAMPLE 46 to 57

A biodegradable pure iron stent is treated by the following processes:
La thin films are deposited onto the pure iron stent surface by plasma deposition process. The film thickness range is from 10 to 1000 nanometer. The parameters for example 46- 57 are given in Table 6.

Table 6 also shows the result of average corrosion rate and APTT, the "average corrosion rate (mm/year) is deduced from the average data from testing for 216 hours. It is proved that the corrosion resistance and anticoagulation properties are significantly increased.

**Table 6**

| | Film thickness (nm) | Grain size (nm) | Average corrosion rate (mm/year) | APTT(s) |
|---|---|---|---|---|
| Controlling sample | Unmodified iron stent | | 0.039 | 39.5 |
| Example 46 | 10 | 10 | 0.028 | 41.5 |
| Example 47 | 200 | 10 | 0.012 | 43.0 |
| Example 48 | 500 | 10 | 0.016 | 42.5 |
| Example 49 | 1000 | 10 | 0.019 | 43.1 |
| Example 50 | 10 | 100 | 0.030 | 41.1 |
| Example 51 | 200 | 100 | 0.019 | 42.5 |
| Example 52 | 500 | 100 | 0.024 | 42.2 |
| Example 53 | 1000 | 100 | 0.028 | 42.5 |
| Example 54 | 10 | 200 | 0.033 | 41.0 |
| Example 55 | 200 | 200 | 0.016 | 41.6 |
| Example 56 | 500 | 200 | 0.021 | 42.7 |
| Example 57 | 1000 | 200 | 0.022 | 42.1 |

### EXAMPLE 58 to 69

A biodegradable pure iron stent is treated by the following processes:
Ce thin films are deposited onto the pure iron stent surface by plasma deposition process. The film thickness range is from 10 to 1000 nanometer. The parameters for example 58-69 are given in Table 7.

Table 7 also gives the results of average corrosion rate and APTT of the surface modified stent. It is proved that the stent with Ce thin film has significantly increased corrosion resistance and anticoagulation properties.

**Table 7**

| | Film thickness (nm) | Grain size (nm) | Average corrosion rate (mm/year) | APTT(s) |
|---|---|---|---|---|
| Controlling sample | Unmodified iron stent | | 0.039 | 39.5 |
| Example 58 | 10 | 10 | 0.033 | 41.0 |
| Example 59 | 200 | 10 | 0.019 | 42.5 |
| Example 60 | 500 | 10 | 0.017 | 42.0 |
| Example 61 | 1000 | 10 | 0.020 | 42.5 |
| Example 62 | 10 | 100 | 0.035 | 41.6 |
| Example 63 | 200 | 100 | 0.018 | 42.0 |
| Example 64 | 500 | 100 | 0.020 | 42.8 |
| Example 65 | 1000 | 100 | 0.019 | 41.5 |
| Example 66 | 10 | 200 | 0.036 | 41.6 |
| Example 67 | 200 | 200 | 0.023 | 41.9 |
| Example 68 | 500 | 200 | 0.018 | 43.6 |
| Example 69 | 1000 | 200 | 0.017 | 41.7 |

### EXAMPLE 70 to 81

A biodegradable pure iron stent is treated by the following processes:
Sr thin films are deposited onto the pure iron stent surface by plasma deposition process. The film thickness range is from 10 to 1000 nanometer. The parameters for example 70 - 81 are given in Table 8.

Table 8 also gives the results of average corrosion rate and APTT of the stent with Sr thin film. It is proved that the stent with Sr thin film has significantly increased corrosion resistance and anticoagulation properties.

**Table 8**

| | Film thickness (nm) | Grain size (nm) | Average corrosion rate (mm/year) | APTT(s) |
|---|---|---|---|---|
| Controlling sample | Unmodified iron stent | | 0.039 | 39.5 |
| Example 70 | 10 | 10 | 0.031 | 41.5 |
| Example 71 | 200 | 10 | 0.023 | 43.2 |
| Example 72 | 500 | 10 | 0.017 | 42.6 |
| Example 73 | 1000 | 10 | 0.019 | 41.6 |
| Example 74 | 10 | 100 | 0.033 | 42.4 |
| Example 75 | 200 | 100 | 0.015 | 43.0 |
| Example 76 | 500 | 100 | 0.017 | 42.6 |
| Example 77 | 1000 | 100 | 0.021 | 41.9 |
| Example 78 | 10 | 200 | 0.035 | 42.1 |
| Example 79 | 200 | 200 | 0.021 | 42.5 |
| Example 80 | 500 | 200 | 0.018 | 42.6 |
| Example 81 | 1000 | 200 | 0.022 | 42.8 |

### EXAMPLE 82 to 93

A biodegradable pure iron stent is treated by the following processes:
Fe thin films are deposited onto the pure iron stent surface by plasma deposition process. The film thickness range is from 10 to 1000 nanometer. The parameters for example 82-93 are given in Table 9.

Table 9 also gives the results of average corrosion rate and APTT of the stent with Fe thin film. It is proved that the stent with Fe thin film has significantly increased corrosion resistance.

**Table 9**

| | Film thickness (nm) | Grain size (nm) | Average corrosion rate (mm/year) |
|---|---|---|---|
| Controlling sample | Unmodified iron stent | | 0.039 |
| Example 82 | 10 | 10 | 0.029 |
| Example 83 | 100 | 10 | 0.014 |
| Example 84 | 1000 | 10 | 0.011 |
| Example 85 | 2000 | 10 | 0.013 |
| Example 86 | 10 | 100 | 0.032 |
| Example 87 | 100 | 100 | 0.021 |
| Example 88 | 1000 | 100 | 0.023 |
| Example 89 | 2000 | 100 | 0.024 |
| Example 90 | 10 | 200 | 0.036 |
| Example 91 | 100 | 200 | 0.033 |
| Example 92 | 1000 | 200 | 0.032 |
| Example 93 | 2000 | 200 | 0.035 |

### EXAMPLE 94 to 105

A biodegradable pure iron stent is treated by the following processes:
Iron oxide thin films are deposited onto the pure iron stent surface by plasma deposition process. The film thickness range is from 10 to 1000 nanometer. The parameters for example 94-105 are given in Table 10.

Table 10 also gives the results of average corrosion rate and APTT. It is proved that the stent with iron oxide thin film has significantly increased corrosion resistance and anticoagulation properties.

**Table 10**

| | Film thickness (nm) | Grain size (nm) | Corrosion current (mA/cm²) | APTT(s) |
|---|---|---|---|---|
| Controlling sample | Unmodified | | 0.58 | 39.5 |
| | iron stent | | | |
| Example 94 | 10 | 10 | 0.45 | 40.5 |
| Example 95 | 100 | 10 | 0.12 | 43.0 |
| Example 96 | 1000 | 10 | 0.17 | 42.5 |
| Example 97 | 2000 | 10 | 0.22 | 43.1 |
| Example 98 | 10 | 100 | 0.47 | 42.0 |
| Example 99 | 100 | 100 | 0.19 | 41.0 |
| Example 100 | 1000 | 100 | 0.25 | 42.6 |
| Example 101 | 2000 | 100 | 0.29 | 41.5 |
| Example 102 | 10 | 200 | 0.49 | 42.0 |
| Example 103 | 100 | 200 | 0.21 | 41.0 |
| Example 104 | 1000 | 200 | 0.26 | 41.5 |
| Example 105 | 2000 | 200 | 0.27 | 40.0 |

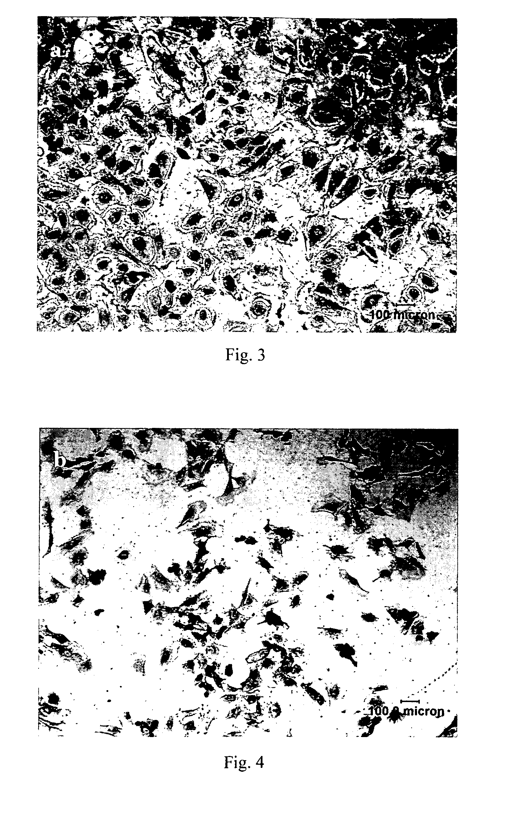

Fig. 3 is surface morphology of the endothelial cells on the stent with iron oxide film described in Example 95 cultured with endothelial cells for 3 days.

Fig. 4 is surface morphology of the endothelial cells on the stainless steel stent cultured with endothelial cells for 3 days.

Comparing Fig. 3 with Fig.4, it shows that endothelial cells grow more on the iron stent with iron oxide film. This means that the biocompatibility of iron oxide film coated stent is better.

The iron stent after surface modification is treated further in magnetic field. The magnetic density is not lower than 100mT. After magnetization the stent is implanted in the blood vessel and it is found that the stent is fully covered with ECs in 2-5 days. In comparison to magnetized stent, ECs covering on non-magnetized stent occurs in more than 10 days. It shows that magnetization of stent can help endothelialization on the iron stent.

### EXAMPLE 106 to 114

A biodegradable iron alloy stent contains La, Ce or Sr from 0.01 to 0.5 atom%. example 106 -114 about the iron alloy stents with different amount of alloying elements are given in Table 11. Polarization testing and immersion corrosion testing are performed in simulated body fluid solution (SBF, containing NaCl:8.04, KCl:0.23, NaHCO3:0.35, K2HPO4·3H2O:0.24, MgCl2·6H2O:0.31, CaCl2:0.29, Na2SO4:0.07, TRIS:6.12) to verify the corrosion and in vitro degradation properties of the stents and the results are also given in Table 11. The test results show that the corrosion currents and the weight loss rate in SBF for 187 hours all are decreased. (The corrosion rate in Table 11 is deduced from data of 187 hours immersion.)

**Table 11**

| | Alloy element | Atom% | Corrosion current (mA/cm²) | Average corrosion rate (mm/year) |
|---|---|---|---|---|
| Controlling sample | | Pure iron | 0.57 | 0.038 |
| Example 106 | La | 0.01% | 0.46 | 0.032 |
| Example 107 | La | 0.25% | 0.31 | 0.023 |
| Example 108 | La | 0.5% | 0.37 | 0.029 |
| Example 109 | Ce | 0.01% | 0.44 | 0.032 |
| Example 110 | Ce | 0.25% | 0.27 | 0.020 |
| Example 111 | Ce | 0.5% | 0.39 | 0.030 |
| Example 112 | Sr | 0.01% | 0.38 | 0.028 |
| Example 113 | Sr | 0.25% | 0.36 | 0.025 |
| Example 114 | Sr | 0.5% | 0.47 | 0.033 |

### EXAMPLE 115 to 117

A biodegradable iron stent contains La, Ce or Sr from 0.01 atom% to 0.5 atom%. Furthermore, oxygen or nitrogen ions are implanted into the stent surface by ion implantation or plasma immersion ion implantation. The doses are from 1×10¹⁶ to 5×10¹⁸ atoms/cm² the energy of the ions are from 5 to 100 KeV.

The treatment conditions and test results of example 115 -117 are given in Table 12.

**Table 12**

| | Controlling sample | Example 115 | Example 116 | Example 117 |
|---|---|---|---|---|
| Alloy content | Not added | La 0.01% | Ce0.25% | Sr0.5% |
| Method for surface treatment | No-treated | Ion implantation | PIII | Ion implantation |
| Element implanted | | O+ | N+ | O+ |
| doses atom/cm² | | 1×10¹⁶ | 5×10¹⁷ | 5×10¹⁸ |
| energy KeV | | 5 | 50 | 100 |
| Corrosion current (mA/cm²) | 0.57 | 0.37 | 0.12 | 0.10 |

### EXAMPLE 118 to 120

A biodegradable iron stent contains La, Ce or Sr from 0.01 to 0.5 atom%. Furthermore, La, Ce or Sr ions are implanted into the stent surface by ion implantation or plasma immersion ion implantation. The doses are from 1×10¹⁶ to 5×10¹⁸ atoms/cm² the energy of the ions are from 5 to 100 KeV.

The treatment conditions and test results of example 118-120 are given in Table 13.

**Table 13**

| | Controlling sample | Example 118 | Example 119 | Example 120 |
|---|---|---|---|---|
| Alloy content | Pure iron | La 0.01% | Ce0.25% | Sr0.5% |
| Method for surface treatment | untreated | Ion implantation | Plasma immersion ion implantation | Ion implantation |
| Element implanted | | La | Ce | Sr |
| doses atom/cm² | | 1×10¹⁶ | 5×10¹⁷ | 5×10¹⁸ |
| energy KeV | | 5 | 50 | 100 |
| Corrosion current (mA/cm²) | 0.57 | 0.39 | 0.23 | 0.20 |

### EXAMPLE 121 to 129

A biodegradable iron stent containing La, Ce or Sr from 0.01 atom % to 0.5 atom%: the stent are further treated by plasma deposition of La, Ce, Sr, lanthana, ceria, strontia, iron or iron oxide thin film. The thickness of the films is from 10 to 1000 nanometers, the grain size is from 10 to 200 nanometers. The treatment conditions and test results of example 121-129 are given in Table 14.

**Table 14**

| | Alloy content | Deposition material | Grain size (nm) | Thickness of the films (nm) | Average corrosion rate (mm/year) |
|---|---|---|---|---|---|
| Controlling sample | Untreat pure iron | | | | 0.039 |
| Example 121 | La 0.01% | La | 10 | 10 | 0.027 |
| Example 122 | Ce 0.25% | Ce | 100 | 700 | 0.016 |
| Example 123 | La 0.01% | La | 200 | 10 | 0.031 |
| Example 124 | Ce 0.25% | strontia | 10 | 500 | 0.015 |
| Example 125 | Sr 0.5% | Ceria | 100 | 1000 | 0.017 |
| Example 126 | Sr 0.5% | Lanthana | 200 | 1000 | 0.020 |
| Example 127 | La 0.01% | Fe | 10 | 10 | 0.030 |
| Example 128 | Ce 0.3% | Iron oxide | 100 | 1000 | 0.028 |
| Example 129 | Sr 0.4% | Fe | 200 | 1000 | 0.029 |

The surface treated degradable iron stent is further magnetized in magnetic field. The magnetic density is not lower than 100mT. After magnetization the stent is implanted in the blood vessel and it is found that the stent is fully covered with ECs in 2-5 days. In comparison to magnetized stent, ECs covering on non-magnetized stent occurs in more than 10 days. This proves that magnetization of stent helps endothelialization of iron stent.

## Claims

1. A degradable stent made of pure iron; doing surface modification using ion implantation and/or plasma surface modification methods, as follows:
Implanting oxygen or nitrogen ions into the pure iron stent surface using ion implantation or plasma immersion ion implantation processes, the doses range is from 1×10¹⁶ to 5×10¹⁸ atoms/cm², the energy range of the ions is from 5 to 100 KeV;
Or implanting lanthanum (La), cerium (Ce) or strontium (Sr) ions into the pure iron stent surface using ion implantation or plasma immersion ion implantation processes, the doses range is from 1×10¹⁶ to 5×10¹⁸ atoms/cm², the energy range of the ions is from 5 to 100 KeV;
Or depositing a thin film with definite material on the pure iron stent surface, the thin film materials are La, Ce, Sr, lanthana, ceria, strontia, iron or iron oxide, The thickness of the films is from 10 to 1000 nanometers, the grain size is from 10 to 200 nanometers.

2. The stent as set forth in claim 1 further magnetization treatment in a magnetic field.

3. A degradable stent made of iron alloy containing lanthanum (La), cerium (Ce) or strontium (Sr) elements with content from 0.01 atom% to 0.5 atom%.

4. The stent as set forth in claim 3 further Surface modification using ion implantation and/or plasma method, as follows:
Implanting oxygen or nitrogen into surface of the iron alloy stent according to Claim 3 using ion implantation or plasma immersion ion implantation processes, the doses range is from 1×10¹⁶ to 5×10¹⁸ atoms/cm², the energy range of the ions is from 5 to 100 KeV;

5. The stent as set forth in claim 3 further Surface modification using ion implantation and/or plasma method, as follows:
implanting lanthanum (La), cerium (Ce) or strontium (Sr) ions into surface of the iron alloy stent according to Claim 3 by ion implantation or plasma immersion ion implantation processes, the doses range is from 1×10¹⁶ to 5×10¹⁸ atoms/cm², the energy range of the ions is from 5 to 100 KeV;

6. The stent as set forth in claim 3 further Surface modification using ion implantation and/or plasma method, as follows:
depositing a thin film on surface of the iron alloy stent according to Claim 3, The thin film materials are La, Ce, Sr, lanthana, ceria, strontia, iron or iron oxides, The thickness of the films is from 10 to 1000 nanometers and the grain size is from 10 to 200 nanometers.

7. The stent as set forth in claim 3 further magnetization treatment in a magnetic field.
